# Europäisches Patentamt
## European Patent Office
### Office européen des brevets

(19)

(11) Veröffentlichungsnummer: **0 181 360**
**B1**

(12) # EUROPÄISCHE PATENTSCHRIFT

(45) Veröffentlichungstag der Patentschrift:
02.08.89

(51) Int. Cl.⁴: **A 61 F 2/30,** A 61 C 8/00

(21) Anmeldenummer: **85902036.4**

(22) Anmeldetag: **04.05.85**

(86) Internationale Anmeldenummer:
**PCT/EP 85/00196**

(87) Internationale Veröffentlichungsnummer:
**WO 85/05026 (21.11.85 Gazette 85/25)**

(54) **IMPLANTAT.**

(30) Priorität: **06.05.84 DE 3416872**
**27.06.84 DE 3423667**

(43) Veröffentlichungstag der Anmeldung:
**21.05.86 Patentblatt 86/21**

(45) Bekanntmachung des Hinweises auf die Patenterteilung:
**02.08.89 Patentblatt 89/31**

(84) Benannte Vertragsstaaten:
**BE CH FR GB IT LI NL SE**

(56) Entgegenhaltungen:
**EP-A-0 023 608**
**EP-A-0 024 008**
**EP-A-0 071 242**
**EP-A-0 094 829**
**DE-A-2 502 884**
**DE-A-2 611 744**
**DE-A-2 620 907**
**DE-A-2 628 284**
**US-A-4 051 598**

(73) Patentinhaber: **Fried. Krupp Gesellschaft mit beschränkter Haftung, Altendorfer Strasse 103, D-4300 Essen 1 (DE)**

(72) Erfinder: **SCHEUNEMANN, Rüdiger, Karpenbachweg 13, D-5204 Lohmar- Donrath (DE)**

(74) Vertreter: **Vomberg, Friedhelm, Dipl.- Phys., Graf-Recke- Strasse 231, D-4000 Düsseldorf 1 (DE)**

EP 0 181 360 B1

## Beschreibung

Die Erfindung betrifft ein Implantat für Knochen- und Zahnwurzelersatz, das aus einem Kern und einer Matrix mit eingelagerten Partikeln besteht. Insbesondere sind auch Gelenkimplantate angesprochen. Die Erfindung betrifft ferner ein Verfahren unter Einsatz des erfindungsgemäßen Implantats.

Es ist bekannt, daß als Knochen- und Gelenkersatz bzw. Zahnwurzelersatz metallische, keramische und Faserverbund-Implantate verwendet werden, bzw. diese sich in der Entwicklung befinden. Die verwendeten Materialien sind teilweise bioniert bzw. teilweise biokompatibel. Es werden in vielen Fällen Beschichtungen mit anorganischen und organischen Substanzen mit bioaktiver Wirkung angestrebt. Diese Beschichtungsmaterialien sind mehr oder minder resorbierbar und spätestens nach einigen Jahren vom Körper aufgelöst. Da das Heranwachsen bzw. Anwachsen des Knochens nur an der Oberfläche des Implantats erfolgt, treten Lockerungserscheinungen bei allen bekannten Implantatmaterialien auf.

Bei Zahnwurzelimplantaten ist nach dem Stand der Technik zwischen Implantat und Kieferknochen eine starre Verbindung möglich, die nicht den natürlichen Bedingungen angepaßt ist. Deshalb sind aufwendige druckspitzendämpfende Konstruktionen bei den derzeitigen Zahnimplantaten erforderlich, die ein Ausbrechen des Implantats aus dem Kieferknochen vermeiden sollen. Aufgrund der falschen Belastung des Kieferknochens kommt es bei handelsüblichen Implantaten auch zu dessen Abbau.

Ferner sind Implantate aus porösen gewebeverträglichen Metallen, Kunststoffasern oder Metallverbindungen bekannt, bei denen in den Randschichten eine Kunststoffmatrix mit eingelagerten anorganischen resorbierbaren Partikeln enthalten ist. Es hat sich jedoch auch bei diesen nach dem Stand der Technik bekannten Implantaten herausgestellt, daß ein störungsfreier Verbund zwischen Knochen und Fasermaterial nicht möglich ist. Die Folge hiervon ist, daß nach einer gewissen Zeit die Implantate gegen neue ausgetauscht werden müssen, was meist bei einer dann notwendigen Zweitoperation ein weiteres Ausräumen des Knochens erfordert, z. B. längere Schäfte oder größere Hüftpfannen.

Aus der EP-A1-0 024 008 ist ein Implantat bekannt, deren mit.dem biologischen Körperteil zu vereinigende Oberfläche mindestens teilweise von Fasermaterial überdeckt ist, dessen Fasern vorzugsweise vornehmlich in derjenigen Richtung verlaufen, in der die Aufhängung des ursprünglichen, zu ersetzenden Körperteils, bzw. dieses selbst auf Zug beansprucht worden wäre. Da die Elastizität des Fasermaterials aber nicht der des das Fasermaterial umlagernden Gewebes entspricht, sind Relativbewegungen zwischen Fasermaterial und umgebenden Gewebe unvermeidbar. Diese Relativbewegungen führen letztlich zu Lockerungen, so daß ein Einwachsen langfristig nicht möglich ist.

Der Erfindung liegt daher die Aufgabe zugrunde, ein Implantat zu schaffen, daß eine dauerhafte bzw. lebenslange Verbindung bzw. Verankerung des Implantats mit dem natürlichen Knochen gewährleistet und damit Wiederholungsoperationen bei Implantationen verringert oder ganz vermeidet.

Die Aufgabe wird durch ein Implantat gelöst, dessen metallischer oder aus Verbundmaterial bestehender Kern knochenseitig mit einer Übergangsstruktur versehen ist, die im Kern dauerhaft verankert, igelstachel- oder bürstenartig - vorzugsweise senkrecht zur Implantatoberfläche auslaufende, Federform aufweisende, flexible metallische Drähte oder flexible Fasern aufweist, die von der Matrix umgeben sind, und daß die Federhärte der Federn dem Elastizitätsmodul des später die Drähte oder Fasern umlagernden Knochengewebes im wesentlichen entspricht.

Dadurch erfüllen die Drähte oder Fasern Knochenverbund auch bei Belastungen aller Art, wie Zug-, Druck-, Biege-, Torsions- und Scherkräften zwei Bedingungen:

1. Vom Faser- bzw. Drahtende bis zum Implantatkern wird eine im wesentlichen gleichmäßige Lastaufnahme über die Länge der Faser oder des Drahts gewährleistet.
2. Es entstehen keine oder nur geringfügige Mikro- oder Relativbewegungen zwischen den Fasern oder den Drähten und dem Knochen oder den Knochenzellen. Dazu wird durch die Drähte bzw. Fasern die Haftfläche zwischen Knochen und Implantat vergrößert.

Nach einer Weiterbildung der Erfindung ist die Matrix knochenseitig ganz oder teilweise resorbierbar, bzw. besteht vorzugsweise ganz oder teilweise aus natürlicher Knochensubstanz. Als Implantatwerkstoff bieten sich körperverträgliche organische, anorganische Stoffe oder Mischungen hiervon an, als resorbierbare Stoffe insbesondere diejenigen, die bioaktiv und/oder osteoinduktiv sind, wie z. B. Trikalzium-Phosphat, Hydroxyl-Apatit und Biogläser.

Durch den Einsatz eines solchen Implantats mit der beschriebenen speziellen Übergangsstruktur und einer bevorzugten knochenseitig teilweise oder ganz resorbierbaren Matrix aus organischen und/oder anorganischen bioaktiven und/oder osteoinduktiven Materialien, die nach der Implantation resorbiert und durch naturliche Knochen ersetzt werden, kann eine dauerhafte (lebenslange) Verbindung bzw. Verankerung des Implantats mit dem natürlichen Knochen gewährleistet werden. Diese Drähte oder Fasern werden vom Knochen, insbesondere bei einer Draht- oder Faserdicke, die kleiner als 800 μm ist, bindegewebsfrei und damit fest eingeschlossen. Hierdurch entsteht ein natürlicher Verbund zwischen Knochen und Implantat. Bevorzugt werden Drähte oder Fasern verwendet, die im wesentlichen gleichmäßige Spannung und Festigkeit über ihre Länge aufweisen.

Die Fasern oder Drähte, die aus einer starren Verankerung bürsten- oder igelstachelartig einzeln oder in Gruppen aus dem Implantatkern heraustreten, werden nach ihrer Implantation vom Knochen bindegewebsfrei ganz oder teilweise eingeschlossen. Bei einer elastischen Verankerung, wie z. B. bei Zahnimplantaten, wird eine Bindegewebsschicht zwischen den Fasern bzw. Drähten gebildet. Z. B. wird beim Zahnwurzelersatz der spezielle Zahnhalteapparat des natürlichen Zahnes durch im E-Modul angepaßte Fasern oder Drähte nachgebildet, die eine elastische Verbindung zwischen dem Zahnwurzelimplantat und dem Kieferknochen nach dem Einwachsen des Kieferknochens um die Übergangsstruktur darstellen. Zwischen dem Implantatkern und dem Knochengewebe wird eine Bindegewebsschicht gebildet.

Die Struktur der Fasern oder Drähte kann spiralförmig oder gewellt sein. In jedem Falle sind die Drähte oder Fasern flexibel und der Steifigkeit des Knochens angepaßt. Wählt man die Spiralenform, so kann nach einer Weiterbildung der Erfindung die Spirale zylindrisch, konisch oder parabelförmig ausgestaltet sein und vorzugsweise eine Steigung besitzen, die gleich oder größer dem Spiraldrahtdurchmesser ist.

Bei zylinderförmigen Spiralen wird die konstante Biegebeanspruchung über die Länge dadurch erreicht, daß die Spiraldrahtdicke zur Spiralspitze hin abnimmt. Bei den konischen oder parabelförmigen Spiralquerschnitten kann die Spiraldrahtdicke ebenfalls variiert werden. Gegebenenfalls bietet es sich zur Aufnahme von Scherkräften noch an, die Spirale mit einer Seele zu versehen, die fest mit dem Implantatkern verbunden ist. Zwischen Spirale und Seele besteht vorzugsweise knochenseitig keine feste Verbindung.

Durch die Spiralmaterialien und Spiralgeometrie werden somit die Elastizitätsmodule zwischen dem Knochengewebe und der Spirale durch die Wahl der Federkonstanten einander angepaßt. Hierdurch ergeben sich bei Belastungen des Knochens und des Implantats, z. B. bei Biegewechselbelastungen, keine Mikrobewegungen zwischen Feder (Spirale) und dem anliegenden Knochen.

Bei den gewellten Strukturen der Fasern oder Drähte wird eine gleichmäßige Festigkeit durch unterschiedliche Fasergeometrien in der Faser selbst bzw. zu den benachbarten Fasern sowie durch unterschiedliche Fasermaterialien erreicht. Die Fasern bzw. Drähte werden vorzugsweise versetzt zueinander angeordnet.

Da in der Regel die spiralförmigen bzw. faserigen Strukturen erst nach dem Einwachsen des Knochengewebes Kräfte aufnehmen und übertragen können, bietet es sich vorzugsweise an, die Zwischenräume zwischen den Drähten oder Fasern mit einem ganz oder teilweise resorbierbaren bioaktiven und/oder osteoinduktiven Material aufzufüllen. Die knochenseitig in das Implantat eingebrachte Substanz wird durch den physiologischen Einfluß ganz oder teilweise resorbiert und durch natürlichen Knochen ersetzt. Je nach Anwendungsart ist ein Auffüllen der Zwischenräume mit natürlicher Knochensubstanz (Spongiosa) möglich. Nach Resorption des Füllmaterials werden die federförmigen Fasern oder Drähte einzeln und störungsfrei vom Knochen umwachsen. Hierdurch wird einerseits die Haftfläche zwischen Knochen und Implantat vergrößert, andererseits beim bindegewebsfreien Einschluß durch den natürlichen Knochen eine Verbindung zwischen ihm und dem Implantat geschaffen, selbst dann, wenn es zu keinem Chemismus zwischen ihnen kommt.

Die Faser- oder Drahtrichtung zur Oberfläche des Implantatlagers wird je nach Verwendungsart angepaßt. Gleichgültig, wie die Fasern oder Drähte aus der Implantatoberfläche heraustreten, ist in jedem Falle eine Überkreuzung der Fasern bzw. Drähte unzulässig, da hierdurch das biologische Wachstum der Knochenzellen gestört wird. Bei Überschneidungen von Fasern bzw. Drähten ist nämlich kein totaler Einschluß gewährleistet, z. B. bei unsystematischer Faseranordnung oder bei herkömmlichen Faserverbundstrukturen, wie sie bei glasfaserverstärkten Kohlenstoffen, kohlenstoffaser-verstärkten Kohlenstoffen und kohlenstoffaser-verstärkten Kunststoffen üblich sind.

Die Fasern oder Drähte können entweder aus metallischen Werkstoffen oder aus organischen bzw. anorganischen Fasern bestehen. Bei metallischen Werkstoffen kann vorgesehen werden, daß die metallischen Fasern über einen galvanischen Prozeß senkrecht zur Oberfläche wachsen. Das Faser- bzw. Drahtmaterial wird hinsichtlich seiner Festigkeit und Elastizität den physiologischen Erfordernissen angepaßt. Die knochenseitig in das Implantat eingebrachte Substanz zwischen den einzelnen Fasern oder Drähten besteht entweder aus anorganischen oder organischen teilweise oder ganz resorbierbaren bioaktiven und/oder osteoinduktiven Materialien. Die Grenzschicht zwischen dem in das Implantat durch Auflösung der Matrix hineinwachsenden Knochen und dem Restimplantat, d.h. dem unauflösbaren Kern aus nicht resorbierbarem Faserverbundmaterial oder metallischen Werkstoffen, kann zusätzlich kernseitig mit einem bioaktiven, einem ganz oder teilweise nicht oder schwer resorbierbaren Material beschichtet werden, um die Haftfähigkeit zwischen natürlichem Knochen und Implantat zu vergrößern. Die bioaktive, nicht oder schwer resorbierbare Grenzschicht kann durch kovalente Bindungen, d.h. durch einen Kleber, eine zusätzliche Verankerung des Knochens an das Implantat erhalten.

Das Implantat soll aufgabengemäß als Langzeitimplantat, d.h. lebenslang, als Knochenersatzwerkstoff dienen. Durch die besondere Verbindung zwischen Knochen und Implantat können die physiologischen Bedingungen insbesondere durch Erhaltung des natürlichen Kraftschlusses und des daraus resultierenden Kraftflusses erreicht werden.

Beim künstlichen Gelenkersatz, z. B. bei der Hüftgelenkspfanne, wird beim Einwachsen des Knochens in das Implantat eine Quasi-Knochenbruchheilung erzielt, d.h. eine feste unbewegliche Verbindung zwischen Knochen und Implantat. Selbst bei Komplikationen, z. B. bei Infektionen und Wiederholungsoperationen, wird das lebende Knochengewebe nicht über das ursprüngliche Maß hinaus geschädigt, da hier für eine Zweitimplantation keine größere Knochenausräumung erfolgen muß.

Bei Zahnwurzelimplantaten wird durch Draht- oder Faserwahl, d.h. Art und Größe, eine auf Zug und Druck belastbare elastische Verbindung zwischen Zahnwurzelimplantat und Kieferknochen erzielt. Daher bietet sich insbesondere bei Zahnwurzelimplantaten, aber auch bei anderen Implantaten, die rein zylindrische Spirale an.

Bei Verwendung einer resorbierbaren Matrix bietet es sich ferner an, in diese Antibiotika einzulagern.

Für die feste Verbindung der Drähte oder Fasern mit dem Kern gibt es verschiedene Möglichkeiten: So können die Drähte oder Fasern insbesondere bis in den Kern hineinreichen oder durch einen Kleber an der Implantatoberfläche befestigt, angeschweißt oder angesintert sein.

Die Fasern werden bevorzugt mit bioaktivem und/oder osteoinduktivem Material vor dem Wickeln oder Verpressen beschichtet.

Vorteilhafterweise verwendet man als resorbierbares und/oder osteoinduktives Material eine hochabgeschreckte Substanz mit extremer Dichte von Gitterbaufehlern.

Die Erfindung ist jedoch nicht nur auf solche Ausführungsformen beschränkt, bei denen der Implantatkern und die Drähte oder Fasern aus ein- und demselben Material oder Material der gleichen Art bestehen. Vielmehr können der Implantatkern und die Drähte oder Fasern auch aus verschiedenen Stoffen bestehen, wie z. B. bevorzugt bei Implantaten mit einem Kunststoffkern und darin fest eingebetteten Drähten oder Fasern.

Bei Zahnimplantaten (Zahnwurzelersatz) kann bis nach Abschluß der Defektheilung die Zahnwurzel mit einer zusätzlichen Verankerung, einer Primärfixierung, zum besseren Einwachsen ausgestattet werden. Vorteilhafterweise wird die Primärfixierung nach Abschluß der Defektheilung, d.h. nach Einschluß der Fasern bzw. Drähte, resorbiert. In einem solchen Fall könnte auf die Beschichtung einer resorbierbaren bioaktiven und/oder osteoinduktiven Substanz verzichtet und es könnten die Zwischenräume zwischen den einzelnen Federn, d.h. Drähten oder Fasern, mit natürlicher Knochensubstanz ausgefüllt werden. Die Primärfixierung kann im einfachsten Fall durch Spangen oder Halteklammern erfolgen, die nach der Defektheilung entfernt werden können.

Je nach Belastungsart kann auf eine Faser gleicher Festigkeit bzw. auf Drähte oder Fasern gleicher Dicke und/oder Länge verzichtet werden, wenn die auftretenden maximalen Belastungen klein sind und keine Mikrobewegungen durch die Fasergeometrie, z. B. bei federartigen Spiralen, möglich sind. Bevorzugt werden die Drähte oder Fasern unterschiedlicher Länge so aufeinander abgestimmt, daß sie bei Belastung über die Länge annähernd gleichmäßig beansprucht werden.

In Ausnahmefällen kann auf eine ganz oder teilweise resorbierbare Matrix aus anorganischem und/oder organischem Material verzichtet werden, insbesondere dann, wenn die Spiralen oder Drähte kurz sind, mit einer Seele ausgestattet sind und trotz der flexiblen Eigenschaften eine Grundsteifigkeit (Eigenstabilität) aufweisen. Bei einfachen geometrischen Fasern, gleichen Durchmessers ist das nicht möglich, da es entweder an der Grundsteifigkeit oder an der Flexibilität fehlt.

Ein störungsfreies Einwachsen zwischen Implantat und Knochen erfordert einen störungsfreien Verbund, der nur dann gewährleistet sein kann, wenn die Fasern sich nicht unkontrolliert gegenüber dem Knochen in ihrer Lage verändern können. Es hat sich auch gezeigt, daß bei Fasern mit gleichem Durchmesser und gleichem Material über ihre gesamte Länge eine erhöhte Belastung an der Einspannung der Faser zum Kern auftritt. Ebenfalls treten Mikrobewegungen zwischen Fasern und Knochen bei Belastungen auf, wenn die E-Module nicht aufeinander abgestimmt sind. Erst recht ist bei Implantaten mit starren Obergangsstrukturen, z. B. Stiften oder übereinander angebrachten Kugeln, aufgrund der unterschiedlichen E-Module mit Mikrobewegungen zwischen Knochen und Implantat zu rechnen und damit langfristig mit einer Zerstörung des festen Verbunds Knochen/Implantat. Diese Nachteile beseitigt die vorliegende Erfindung.

Ausführungsbeispiele der Erfindung sind in den Zeichnungen dargestellt. Es zeigen

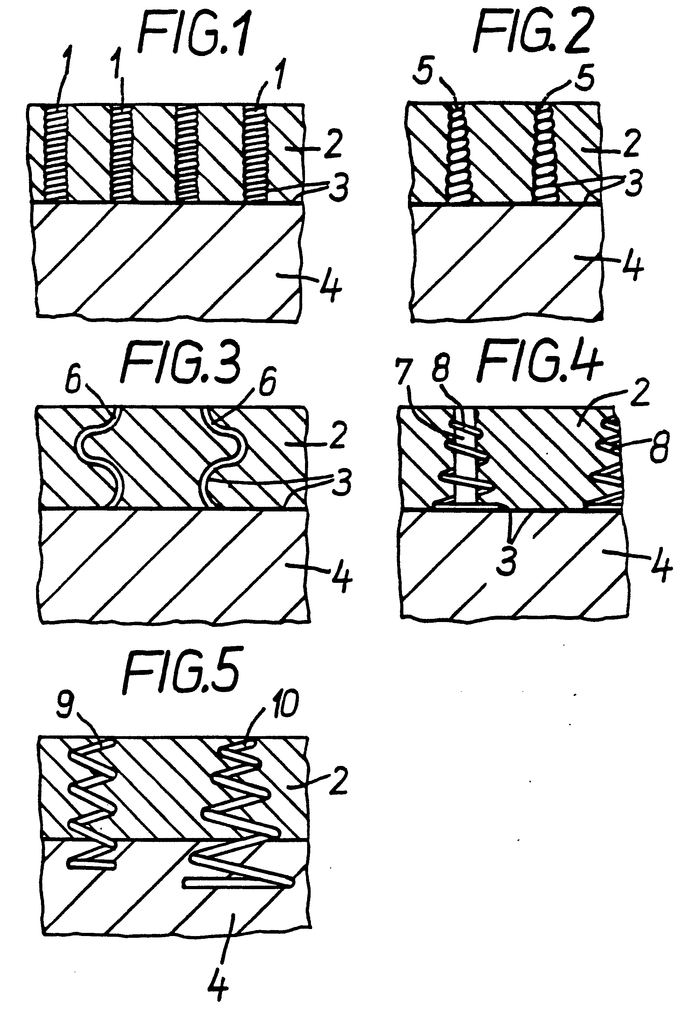

Fig. 1 bis 5 jeweils einen Ausschnitt aus einem Implantat einschließlich seiner aus der Matrix austretenden Fasern oder Drähte.

Die in den genannten Figuren dargestellten Implantate für Knochen- und Zahnwurzelersatz mit spezieller Übergangsstruktur zwischen Knochen und Implantatkern sowie knochenseitig teilweise oder ganz resorbierbarer Matrix 2 bestehen im wesentlichen aus einem Kern 4 aus metallischem Werkstoff oder Faserverbundmaterial sowie aus in einer Matrix 2 befindlichen, dauerhaft verankerten, igelstachel- oder bürstenartig, vorzugsweise senkrecht zur Implantatoberfläche auslaufenden metallischen Drähten oder Verbundmaterialfasern.

So zeigt Fig. 1 einen metallischen Kern 4 sowie an dessen Oberfläche befestigte zu einer engen Spirale gewundene Metalldrähte 1, die an einer mit Kleber beschichteten Grenzschicht 3 an der Kernoberfläche befestigt sind. Der Zwischenraum 2 (die Matrix) ist mit einem ganz oder teilweise resorbierbaren bioaktiven und/oder osteoinduktiven Material aufgefüllt.

Demgegenüber sind bei der Ausführungsform gemäß Fig. 2 die Spiralen 5 konisch geformt. Bei beiden Ausführungsformen nach Fig. 1 und 2 sind Kern 4 und die Drähte einheitlich aus einem einzigen Material, im vorliegenden Fall Metall, gebildet.

Im Unterschied dazu liegen bei den Ausführungsformen nach Fig. 3 bis 5 unterschiedliche Materialien vor, nämlich zum einen Metall und zum anderen Verbundmaterial.

So sind bei dem in Fig. 3 dargestellten Implantat an einem metallischen Kern spiralförmige Verbundmaterialfasern 6 befestigt. Das Implantat gemäß Fig. 4 weist zusätzlich noch eine Seele 7 auf, die von einer Verbundmaterialspirale 8 mit abnehmendem Spiralradius umgeben ist. Diese Spirale 8 sowie die Seele 7 sind beide ebenfalls an der Implantatkernoberfläche befestigt.

Es ist sowohl möglich, die Seele 7 und die sie umhüllende Spirale 8 aus gleichem oder verschiedenem Material zu gestalten, wobei jeweils Metalle oder Verbundmaterialien in Frage kommen.

Das in Fig. 5 dargestellte Implantat besitzt spiralförmige Metalldrähte 9 und 10, wobei im erstgenannten Fall der Spiralradius gleichbleibend ist und im zweiten Fall mit wachsendem Abstand von dem Implantatkern 4 kleiner wird. Es ist daneben auch möglich, Spiralen mit jeweils unterschiedlichem Durchmesser zu verwenden oder auch solche, deren Steigung größer oder gleich dem jeweiligen Spiraldurchmesser ist. Insbesondere reichen die Metalldrähte 9 und 10 bis weit in den Implantatkern 4 aus Verbundmaterial hinein.

Gleichgültig, ob die Drähte oder Fasern in Fig. 1 bis 5 in gleichmäßigen oder ungleichmäßigen Abständen, einzeln oder in Gruppen aus der Implantatoberfläche herausstehen, wird deren Abstand so gewählt, daß eine Versorgung des Knochengewebes zwischen ihnen störungsfrei erfolgen kann.

**Patentansprüche**

1. Implantat für Knochen- und Zahnwurzelersatz, insbesondere auch Gelenkimplantate, bestehend aus einem Kern und einer Matrix mit eingelagerten resorbierbaren Partikeln, dadurch gekennzeichnet, daß der metallische oder aus Verbundmaterial bestehende Kern des Implantates knochenseitig mit einer Übergangsstruktur versehen ist, die im Kern dauerhaft verankert, igelstachel- oder bürstenartig - vorzugsweise senkrecht zur Implantatoberfläche - auslaufende, Federform aufweisende, flexible metallische Drähte oder flexible Fasern aufweist, die von der Matrix umgeben sind, und daß die Federhärte der Federn dem Elastizitätsmodul des später die Drähte oder Fasern umlagernden Knochengewebes im wesentlichen entspricht.

2. Implantat nach Anspruch 1, dadurch gekennzeichnet, daß die Matrix knochenseitig ganz oder teilweise resorbierbar ist, vorzugsweise ganz oder teilweise aus natürlicher Knochensubstanz besteht.

3. Implantat nach Ansprüchen 1 und 2, gekennzeichnet durch körperverträgliche organische oder anorganische Stoffe oder Mischungen hiervon.

4. Implantat nach einem der Ansprüche 1 bis 3, gekennzeichnet durch eine ganz oder teilweise resorbierbare Matrix aus bioaktivem und/oder osteoinduktivem Material, insbesondere Trikalzium-Phosphat, Hydroxyl-Apatit und Biogläser.

5. Implantat nach einem der Ansprüche 1 bis 4, dadurch gekennzeichnet, daß die resorbierbare Matrix Antibiotika enthält.

6. Implantat nach Anspruch 1, dadurch gekennzeichnet, daß die Drähte oder Fasern im wesentlichen eine gleichmäßige Spannung und Festigkeit über ihre Länge aufweisen.

7. Implantat nach einem der Ansprüche 1 bis 6, dadurch gekennzeichnet, daß die Drähte oder Fasern in ungleichmäßigem Abstand voneinander, insbesondere gruppenweise aus der Implantatoberfläche heraustreten.

8. Implantat nach einem der Ansprüche 1 bis 7, dadurch gekennzeichnet, daß der Abstand der Drähte oder Fasern so groß ist, daß eine Versorgung des Knochengewebes zwischen den einzelnen Drähten oder Fasern störungsfrei erfolgen kann.

9. Implantat nach einem der Ansprüche 1 bis 8, dadurch gekennzeichnet, daß Drähte oder Fasern einen gleichen Durchmesser aufweisen.

10. Implantat nach einem der Ansprüche 1 bis 8, dadurch gekennzeichnet, daß die Draht- bzw. Faserdicke und/oder -länge unterschiedlich ist.

11. Implantat nach einem der Ansprüche 1 bis 10, dadurch gekennzeichnet, daß die Drähte oder Fasern eine gewellte Federform besitzen und vorzugsweise versetzt zueinander angeordnet sind.

12. Implantat nach einem der Ansprüche 1 oder 6 bis 10, dadurch gekennzeichnet, daß die Drähte oder Fasern die Form einer Spiralfeder aufweisen, die im Längsschnitt zylindrisch, konisch oder parabelförmig ausgestaltet ist, wobei die Steigung der Spirale gleich oder größer dem Spiraldurchmesser ist.

13. Implantat nach Anspruch 1, dadurch gekennzeichnet, daß die Drähte oder Fasern zur Aufnahme von Scherkräften mit einer Seele versehen sind, die vorzugsweise fest mit dem Implantatkern verbunden ist.

14. Implantat nach einem der Ansprüche 1 bis 14, dadurch gekennzeichnet, daß die Draht- oder Faserdicke kleiner als 800 μm ist.

15. Implantat nach Anspruch 1, dadurch gekennzeichnet, daß die Fasern vor dem Wickeln oder Verpressen mit bioaktivem und/oder osteoinduktivem Material beschichtet worden sind.

16. Implantat nach Anspruch 1, dadurch gekennzeichnet, daß die Drähte oder Fasern fest mit dem Kern verbunden sind.

17. Implantat nach Anspruch 16, dadurch gekennzeichnet, daß die Drähte oder Fasern bis in den Kern hineinreichen.

18. Implantat nach Ansprüche 1 und 16, dadurch gekennzeichnet, daß die Drähte oder Fasern durch einen Kleber an der Implantatoberfläche befestigt oder angeschweißt oder angesintert sind.

19. Implantat nach einem der Ansprüche 1 bis 18, dadurch gekennzeichnet, daß der Kern und die Drähte oder Fasern aus verschiedenen Stoffen bestehen, vorzugsweise die Drähte oder Fasern fest in einem Kunststoffkern eingebettet sind.

20. Implantat nach Ansprüchen 1 und 19, dadurch gekennzeichnet, daß eine Grenzschicht zwischen dem Implantat(kern) und der resorbierbaren Matrix mit einem ganz oder teilweise nicht oder schwer resorbierbaren, bioaktiven und/oder osteoinduktiven Material beschichtet ist.

21. Implantat nach Anspruch 20, dadurch gekennzeichnet, daß die Grenzschicht mit einem Kleber zur kovalenten Bindung des Kerns und der Matrix versehen ist.

22. Implantat nach einem der Ansprüche 1 bis 21, gekennzeichnet durch ein resorbierbares und/oder osteoinduktives Material aus einer hochabgeschreckten Substanz mit extremer Dichte von Gitterbaufehlern.

23. Implantat nach einem der Ansprüche 1 bis 22, insbesondere für den Zahnwurzelersatz, dadurch gekennzeichnet, daß zusätzlich eine Primärfixierung zum besseren Einwachsen angebracht ist.

24. Implantat nach Anspruch 23, dadurch gekennzeichnet, daß die Primärfixierung nach Abschluß der Defektheilung, d.h. nach Einschluß der Fasern bzw. Drähte durch Knochenzellen, resorbiert wird.

25. Implantat nach Anspruch 23, gekennzeichnet durch Spangen oder Halteklammern als Primärfixierung, die nach der Defektheilung entfernt werden können.

26. Implantat als Zahnwurzelersatz nach Ansprüchen 1 bis 15 und 19 bis 25, dadurch gekennzeichnet, daß der spezielle Zahnhalteapparat des natürlichen Zahnes durch im E-Modul angepaßte Fasern und/oder Drähte nachgebildet wird, die eine elastische Verbindung zwischen dem Zahnwurzelimplantat und dem Kieferknochen nach dem Einwachsen des Kieferknochens um die Übergangsstruktur darstellen, so daß zwischen Implantatkern und Knochengewebe eine Bindegewebsschicht gebildet werden kann.

## Claims

1. An implant to replace bones and tooth roots, more particularly including joint implants, comprising a core and a matrix with embedded resorbable particles, characterized in that the implant core, consisting of metal or a composite material, has on the bone side a transitional structure having spring-like flexible metal wires or flexible fibres which are permanently anchored in the core, preferably extend after the fashion of hedgehog bristles or a brush perpendicularly to the implant surface and are enclosed by the matrix, the elastic temper of the springs substantially corresponding to the modulus of elasticity of the bone tissue subsequently enclosing the wires or fibres.

2. An implant according to claim 1, characterized in that the matrix, which can be wholly or partially resorbed on the bone side, preferably consists wholly or partially of natural bone substance

3. An implant according to claims 1 and 2, characterized by organic or inorganic substances or mixtures thereof which are compatible with the body.

4. An implant according to any of claims 1 to 3, characterized by a wholly or partially resorbable matrix of bioactive and/or osteoinductive material, more particularly tricalcium phosphate, hydroxyl apatite and bioglasses.

5. An implant according to any of claims 1 to 4, characterized in that the resorbable matrix contains antibiotics.

6. An implant according to claim 1, characterized in that the wires or fibres have a substantially uniform stressing and strength over their length.

7. An implant according to any of claims 1 to 6, characterized in that the wires or fibres project from the implant surface, more particularly in groups, at different distances from one another.

8. An implant according to any of claims 1 to 7, characterized in that the distance between the wires or fibres is large enough for bone tissue to be supplied unimpeded between the individual wires or fibres.

9. An implant according to any of claims 1 to 8, characterized in that the wires or fibres have an identical diameter.

10. An implant according to any of claims 1 to 8, characterized in that the wires or fibres have different thicknesses and/or lengths.

11. An implant according to any of claims 1 to 10, characterized in that the wires or fibres have an undulating spring shape and are preferably disposed offset in relation to one another.

12. An implant according to any of claims 1 or 6 to 10, characterized in that the wires or fibres have the shape of a spiral spring which is constructed cylindrical, conical or parabolic in longitudinal section, the pitch of the spiral being equal to or greater than the spiral diameter.

13. An implant according to claim 1, characterized in that to absorb shearing forces, the wires or fibres have a stem which is preferably rigidly connected to the implant core.

14. An implant according to any of claims 1 to 13, characterized in that the thickness of the wires or fibres is less than 800 μm.

15. An implant according to claim 1, characterized in that prior to winding or pressing, the fibres are coated with bioactive and/or osteoinductive material.

16. An implant according to claim 1, characterized in that the wires or fibres are rigidly connected to the core.

17. An implant according to claim 16, characterized in that the wires or fibres extend into the core.

18. An implant according to claims 1 and 16, characterized in that the wires or fibres are attached to the implant surface by gluing or welding or sintering.

19. An implant according to any of claims 1 to 18, characterized in that the core and the wires or fibres are made of different materials, the wires or fibres being preferably firmly embedded in a plastics core.

20. An implant according to claims 1 and 19, characterized in that a boundary layer between the implant (core) and the resorbable matrix is coated with a wholly or partially non-resorbable or difficultly resorbable bioactive and/or osteoinductive material.

21. An implant according to claim 20, characterized in that the boundary layer has a glue for the covalent bonding of the core and the matrix.

22. An implant according to any of claims 1 to 21, characterized by a resorbable and/or osteoinductive material of a heavily chilled substance having an extreme density of lattice defects.

23. An implant according to any of claims 1 to 22, more particularly for taking the place of tooth roots, characterized in that a primary fixing is also provided to improve growing-in.

24. An implant according to claim 23, characterized in that when the healing of the defects is completed - i.e., the fibres or wires have been enclosed by bone cells - the primary fixing is resorbed.

25. An implant according to claim 23, characterized by the primary fixing being produced by clasps or retaining clamps which can be removed after the healing of the defects.

26. An implant to take the place of tooth roots according to claims 1 to 15 and 19 to 25, characterized in that the special tooth-retaining apparatus of the natural tooth is imitated by fibres and/or wires of adapted modulus of elasticity which represent a resilient connection between the tooth root implant and the jaw bone after the jaw bone has grown in around the transitional structure, so that a layer of connective tissue can be formed between the implant core and the bone tissue.

**Revendications**

1. Implant pour le remplacement des os et des racines de dents, notamment également pour des articulations, constitué par un noyau et par une matrice à laquelle sont incorporées des particules qui peuvent être résorbées, caractérisé en ce que le noyeau de l'implant, qui est constitué par du métal ou par un matériau composite, comporte, du côté de l'os, une structure de transition qui, durablement ancrée dans le noyau, comporte des fils métalliques souples ou des fibres souples, ayant l'aspect de ressorts, qui sortent de préférence perpendiculairement de la surface du noyau à la manière de piquants de hérisson ou de soies de brosse et qui sont entourés par la matrice et en ce que la dureté élastique des ressorts correspond sensiblement au module d'élasticité du tissu osseux qui entoure ultérieurement les fils métalliques ou les fibres.

2. Implant selon la revendication 1, caractérisé en ce que, du côté de l'os, la matrice peut se résorber entièrement ou partiellement et est constituée de préférence entièrement ou partiellement par une substance osseuse naturelle.

3. Implant selon les revendications 1 et 2, caractérisé en ce qu'il comporte des substances organiques ou minérales compatibles avec l'organisme ou des mélanges de ces substances.

4. Implant selon l'une des revendications 1 à 3, caractérisé en ce qu'il comporte une matrice entièrement ou partiellement résorbable constituée par un matériau bioactif et/ou ostéoinductif, notamment du phosphate tricalcique, de l'hydroxyl-apatite et des verres biologiques.

5. Implant selon l'une des revendications 1 à 4, caractérisé en ce que la matrice résorbable contient des antibiotiques.

6. Implant selon la revendication 1, caractérisé en ce que les fils métalliques ou les fibres ont sur toute leur longueur une tension et une solidité uniformes.

7. Implant selon l'une des revendications 1 à 6, caractérisé en ce que les fils métalliques ou les fibres sortent de la surface de l'implant à des distances irrégulières les uns des autres, notamment par groupes.

8. Implant selon l'une des revendications 1 à 7, caractérisé en ce que la distance entre les fils métalliques ou les fibres est assez grande pour permettre une alimentation normale du tissu osseux entre les différents fils métalliques ou fibres.

9. Implant selon l'une des revendications 1 à 8, caractérisé en ce que les fils métalliques ou les fibres ont le même diamètre.

10. Implant selon l'une des revendications 1 à 8, caractérisé en ce que les épaisseurs et/ou les longueurs des fils métalliques ou des fibres sont différentes.

11. Implant selon l'une des revendications 1 à 10, caractérisé en ce que les fils métalliques ou fibres ont la forme de ressorts ondulés et sont de préférence décalés les uns par rapport aux autres.

12. Implant selon l'une des revendications 1 ou 6 à 10, caractérisé en ce que les fils métalliques ou fibres ont la forme de ressorts en spirale de section longitudinale cylindrique, conique ou parabolique, le pas de la spirale étant égal ou supérieur au diamètre de la spirale.

13. Implant selon la revendication 1, caractérisé en ce que les fils métalliques ou fibres comportent, pour l'absorption des efforts de cisaillement, une âme qui, de préférence, est solidaire du noyau de l'implant.

14. Implant selon l'une des revendications 1 à 14, caractérisé en ce que l'épaisseur des fils métalliques ou des fibres est inférieur à 800 μm.

15. Implant selon la revendication 1, caractérisé en ce qu'avant leur enroulement ou leur compression les fibres sont recouvertes d'un matériau bioactif et/ou ostéoinductif.

16. Implant selon la revendication 1, caractérisé en ce que les fils métalliques ou fibres sont solidaires du noyau.

17. Implant selon la revendication 16, caractérisé en ce que les fils métalliques ou fibres pénètrent dans le noyau.

18. Implant selon les revendications 1 et 16, caractérisé en ce que les fils métalliques ou fibres sont fixés à la surface de l'implant par un adhésif, par soudage ou par frittage.

19. Implant selon l'une des revendications 1 à 18, caractérisé en ce que le noyau et les fils métalliques ou fibres sont constitués par des substances différentes et en ce que les fils métalliques ou les fibres sont de préférence enrobés solidement dans un noyau en matière métallique.

20. Implant selon les revendications 1 et 19, caractérisé en ce qu'une couche limite située entre (le noyau de) l'implant et la matrice résorbable est recouverte d'un matériau bioactif et/ou ostéoinductif qui ne peut pas ou ne peut être que partiellement ou difficilement résorbé.

21. Implant selon la revendication 20, caractérisé en ce que la couche limite comporte un adhésif pour la liaison covalente du noyau et de la matrice.

22. Implant selon l'une des revendications 1 à 21, caractérisé en ce qu'il comporte un matériau résorbable et/ou ostéoinductif constitué par une substance brusquement refroidie comportant une forte proportion de défauts de réseau.

23. Implant selon l'une des revendications 1 à 22, notamment pour le remplacement de racines de dents, caractérisé en ce qu'il comporte une fixation primaire pour faciliter la croissance.

24. Implant selon la revendication 23, caractérisé en ce que la fixation primaire est résorbée après l'élimination du défaut, c'est-à-dire après l'enrobage des fibres ou des fils métalliques par les cellules osseuses.

25. Implant selon la revendication 23, caractérisé par des broches ou des pinces de serrage constituant le dispositif de fixation primaire et pouvant être enlevées après la suppression du défaut.

26. Implant pour le remplacement des racines de dents selon les revendications 1 à 15 et 19 à 25, caractérisé en ce que l'appareil spécial de maintien de la dent naturelle est simulé par des fibres et/ou des fils métalliques de module élastique convenable qui constituent une liaison élastique entre l'implant de la racine de dent et l'os maxillaire après la croissance de l'os maxillaire autour de la structure de transition, ce qui permet la formation d'une couche de liaison entre le noyau de l'implant et le tissu osseux.

FIG.1

FIG.2

FIG.3

FIG.4

FIG.5